# EUROPEAN PATENT APPLICATION

(11) **EP 2 083 258 A1**
(43) Date of publication of application: **29.07.2009**
(21) Application number: 09000634.7
(22) Date of filing: 17.01.2009
(51) Int. Cl.: G01N 3/22, G01N 3/24

(54) **The device for determining the quality and solidness of the vascular wall**

(30) Priority: 25.01.2008 CZ 200819621 U
(71) Applicant: Benedik, Jaroslav, 61300 Brno (CZ)
(72) Inventor: Benedik, Jaroslav, 61300 Brno (CZ)
(74) Representative: Langrova, Irena

(57) **Abstract**

The device can be used to determine the mutual cohesiveness of individual layers of the vascular wall by a destructive method. The device consists of two mutually flexible moving and opposite fixation plates (1 and 2) of random shape. The tissue sample (6) is located between them and is fixed to them. The device is connected to the detector (7) for measuring and recording the value corresponding to the solidness of the tissue sample (6) with mutual abduction or rotation of the fixation plates (1,2).

The inner areas of the fixation plates (1 and 2) can be equipped with holes (3). The holes (3) are interconnected with outlets (4) connected to the air pump creating negative pressure for fixing the tissue sample (6) to fixation plates (1 and 2).

The fixed tissue sample (6) is tested during the movement of the fixation plates (1,2).

## Description

### Technical Field

The technical solution applies to the device to determine the quality and solidness of the vascular wall. The device belongs to a technical solution of devices used during surgical procedures and in research of human tissue or tissue of a different organism.

### Background Art

The principle of determining the quality of arterial tissue is currently determined by imperfect technical solutions. The solution described in file WO 2007/117782 deals with determining the quality of tissue and use of various sources for the treatment of this tissue. According to it the probe detects changes in tissue with the introduction of various types of radiation to this tissue, and subsequently examines changes of radiation during passage through this tissue. The disadvantage of the described solution lies in the fact that it does not directly test the quality (solidness) of tissue and therefore does not define the properties and quality of the tissue.

A further known technical solution examining the tissue of living organisms is described in the valid Czech patent No. 292284. However, the described equipment examines a different nature of the tissue, i.e. its viscosity and therefore does not testify to the properties of the tissue with regard to the risk of dissection of the vascular wall.

### Disclosure of Invention

The core of the proposed technical solution is an device for determining the quality and solidness of the vascular wall in a destructive way. The device is connected to an air pump or other suction device giving rise to negative pressure on the surface of fixation plates in the space between the fixation plates. A sample of the vascular wall measuring several mm² to several tens of mm² inserted in the space between the fixation plates is fixed with the aid of negative pressure by suction to their surface. The layout of moving fixation plates enables the stretching of the tested sample of the vascular wall by their mutual movement up to the point where the internal links are broken between individual layers of the vascular wall. The force required for this disruption is measured and recorded by a detector.
The advantage of the proposed solution is the possibility to comprehensively decide from the information ascertained about preventive cardio-surgery or to extend the existing surgical procedure. Unlike the known solutions the proposed device need not be used in a strictly sterile environment because the tissue is examined outside the patient's body and the used tissue is not returned to the body.
The key factor is to gain current values applying to the quality of the vascular wall of a certain patient and the possibility of making a comparison with standard values and the possibility to decide whether the surgical procedure will prove beneficial or is unnecessary.

### Brief Description of Drawings

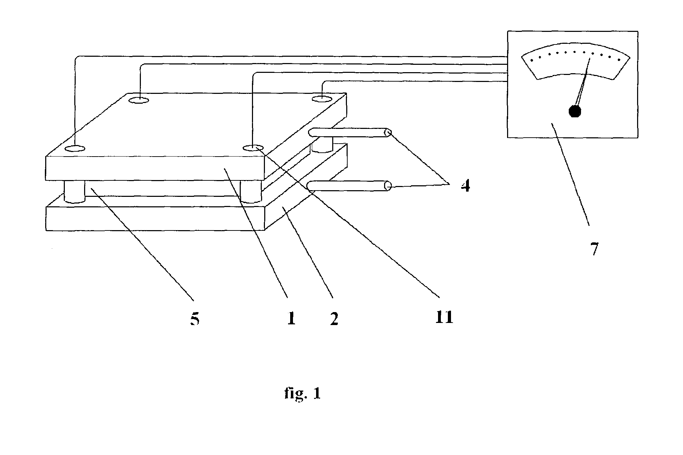

The exemplary version of the proposed solution is described with reference to the drawings in which on
fig. 1 there is an oblique view of the device with linearly abducting square fixation plates and charts indicating the connection to the detector.

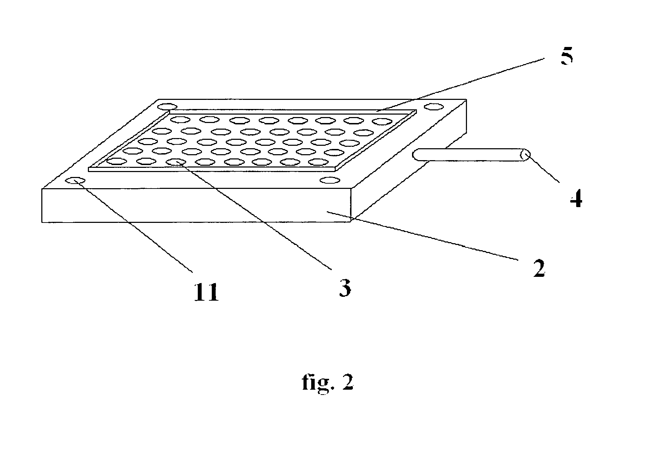

fig. 2 - the oblique view of the bottom fixation plate described in fig. 1 revealing the arrangement of the blade and holes.
fig. 3 - the oblique view of the device with the angular folded back fixation plate and chart indicating the connection to the detector.
fig. 4 - the oblique view of the version of the device with circular fixation plates and rotating top fixation plate.

### Best Mode for Carrying Out the Invention

### Example 1

A device has been constructed for testing the quality of the vascular wall according to the proposed solution. The device consists of two fixation plates 1 and 2. The bottom fixation plate 2 is equipped with a square-shaped blade 5, while the top fixation plate 1 is equipped with a groove 9 corresponding in shape and size with blade 5. Both fixation plates 1 and 2 are hollow inside with a set of channels for the equal distribution of negative pressure directed into holes 3. Holes 3 are directed into the space between the fixation plates 1 and 2 and are situated in the areas defined by the blade 5 and groove 9. These holes 3 allow under-negative pressure to be passed from the outlets 4 to the surface of the fixation plates 1 and 2, and thereby make sure sample 6 of the aortic wall is fixed. The device also consists of an abductor 11 enabling the abduction of the fixation plates 1 and 2. In this case the fixation plates are abducted linearly and the abductor 11 for abducting the fixation plates 1 and 2 is designed and equipped here with four screws. The force required for abducting the fixation plates 1 and 2 is measured and recorded by detector 7 which is connected to the abductor 11 enabling the abduction of the fixation plates 1 and 2. The described version can be seen in figures no. and 2.

### Example 2

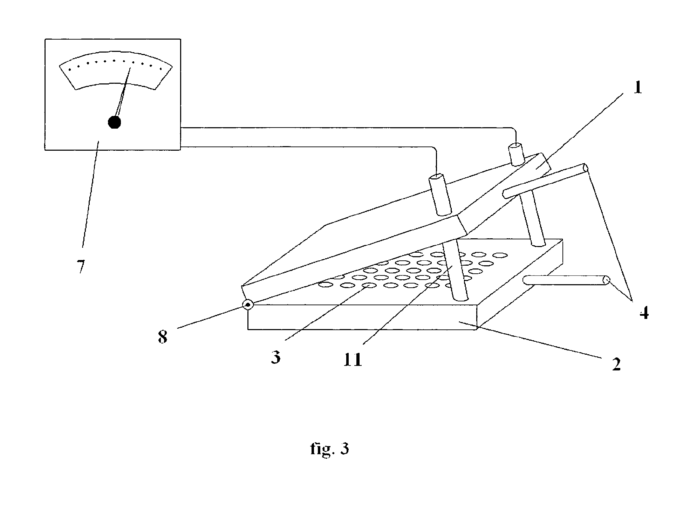

A device has been constructed for testing the quality of the vascular wall according to the proposed solution. The device consists of two fixation plates 1 and 2 connected with the aid of a longitudinal joint 8. The fixation plates 1 and 2 are square shaped with a 20 mm side. Both fixation plates 1 and 2 are hollow inside and a set of holes 3 is directed into the space between them. These holes 3 allow negative pressure to be fed from outlets 4 on the surface of fixing plates 1 and 2, and thereby ensuring fixation of sample 6. The device also consists of an abductor 11 enabling the abduction of the fixation plates 1 and 2. This abduction can be performed smoothly as well as in steps with defined time delays. The force required to abduct the fixation plates 1 and 2 is measured and recorded by a detector 7 which is connected to the abductor 11 enabling abduction of the fixation plates 1 and 2. The described version can be seen in figure no. 3.

### Example 3

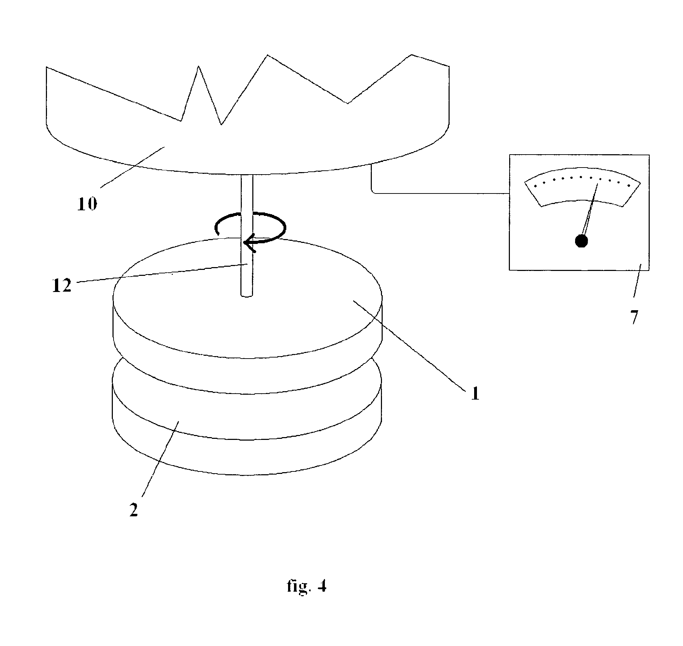

A device has been constructed for testing the quality of the vascular wall according to the proposed solution. The device consists of two fixation plates 1 and 2, in this case circular shaped with a diameter of 15 mm. The fixation of tissue sample 6 in this case is secured with glue due to the small surface size when fixation by negative pressure would prove ineffective. Tissue sample 6 was, in this case, adapted to the necessary shape and surface size apart from the described device. The device also consists of a device rotating the top fixation plate 1 with the aid of shaft 12; in this case this rotation takes place by a step electric motor 10. The force required to abduct the individual layers of tissue in the sample and thereby starting the top fixation plate 1, is measured and recorded with the aid of detector 7. The described solution is seen in figure no. 4.

### Industrial Applicability

The proposed solution can be practically utilised in cardio-surgical operating theatres for all surgical procedures where, based on the testing of the quality of the aortic wall, any surgical indication during the actual procedure will be extended by a procedure carried out on the ascending aorta.

### List of Reference Symbols

1 - top fixation plate
2 - bottom fixation plate
3 - holes
4 - outlet
5 - blade
6 - tissue sample
7 - detector
8 - longitudinal joint
9 - groove
10 - step electric motor
11 - abductor
12 - shaft

## Claims

1. The device for determining the quality and solidness of the vascular wall which can be used to determine the mutual cohesiveness of individual layers of the vascular wall by a destructive method **characterised in that** it consists of two mutually flexible moving and opposite fixation plates (1) and (2) of random shape, while the tissue sample (6) is located between them and is fixed to them, the device is connected to the detector (7) for measuring and recording the value corresponding to the solidness of the tissue sample (6) with mutual abduction or rotation of the fixation plates (1), (2).

2. The device for determining the quality and solidness of the vascular wall according to claim 1 **characterised in that** the fixation plates (1) and (2) are being abducted from each other linearly with the aid of an abductor (11).

3. The device for determining the quality and solidness of the vascular wall according to claim 1 **characterised in that** the fixation plates (1) and (2) are connected by the longitudinal joint (8) for determining their angular abduction with the aid of an abductor (11).

4. The device for determining the quality and solidness of the vascular wall according to claim 1 **characterised in that** the fixation plates (1) and (2) move against each other rotationally with the aid of torsional force, while shaft (12), which is firmly fixed in the top fixation plate (1), passes through the top fixation plate (1) and shaft (12) also passes through the tested sample (6) and is fixed in a slide bearing in the bottom fixation plate (2).

5. The device for determining the quality and solidness of the vascular wall according to claims 1 to 4 **characterised in that** the opposite walls of the fixation plates (1) and (2) are equipped with a blade (5) of a closed geometric shape and groove (9) in shape and size corresponding to the blade (5) for determining the standardised size of the tested sample whose surface area is adapted to the shape of the blade (5) and groove (9).

6. The device for determining the quality and solidness of the vascular wall according to claims 1 to 5 **characterised in that** the inner areas of the fixation plates (1) and (2) are equipped with holes (3) and the holes (3) are interconnected with outlets (4) connected to the air pump creating negative pressure for fixing the tissue sample (6) to fixation plates (1) and (2).
